# EUROPEAN PATENT APPLICATION

(11) **EP 1 040 825 A1**
(43) Date of publication of application: **04.10.2000**
(21) Application number: 99200747.6
(22) Date of filing: 11.03.1999
(51) Int. Cl.: A61K 31/12, A61K 49/00, A61K 9/08

(54) **Means for diagnosing bladder carcinoma**

(71) Applicant: K.U. LEUVEN RESEARCH & DEVELOPMENT, 3000 Leuven (BE)
(72) Inventor: De Witte, Peter Alfred Maria, 3010 Leuven (BE); Waelkens, Etienne Jean Pierre Marie, 3110 Rotselaar (BE); D'Hallewin, Marie-Ange, 3360 Bierbeek (BE); Baert, Luc, 3001 Heverlee (BE)
(74) Representative: Van Someren, Petronella F. H. M.

(57) **Abstract**

The present invention relates to the use of hypericin or analogues or derivatives thereof for the preparation of a diagnostic composition for diagnosing carcinoma, more in particular carcinoma originating from urothelium, especially carcinoma of the bladder. In addition the invention provides an instillation fluid comprising hypericin or analogues or derivatives thereof for use in a method for diagnosing such carcinomas.

## Description

The present invention relates to the diagnosis of carcinoma, in particular of carcinoma of the urothelium with a special interest of carcinoma in situ of the bladder.

Cancer is one of the most common causes of death in the world. Bladder cancer is the sixth most common malignant disease worldwide. When first diagnosed, 75 to 85% of the patients have superficial tumors. More than 70% of patients with superficial bladder tumors will have one or more recurrences after initial therapy and in about one third tumor progression is observed. The cause for this high recurrence rate is the fact that small papillar lesions can be overlooked. Progression is mostly due to the presence of flat carcinoma in situ.

Carcinoma in situ (CIS) of the bladder is a confounding disease that is difficult to recognize clinically as well as histopathologically. Many studies have suggested its relationship with subsequent invasive disease. A study of natural history of untreated CIS has been reported by Wolf et al. (Wolf et al., Scand. J. Urol. Nephrol. Suppl. 157:147-151 (1994)). Of the 31 study patients with a mean follow up of more than 5 years, 52% had tumor progression to invasive disease and 48% has stable disease. Only 3 from the patients with stable disease had no tumor recurrence during follow up. Early recognition of carcinoma in situ is essential in order to offer the patients the most appropriate treatment and the highest cure rate. Since white light cystoscopic examination is not sufficient to reveal areas of dysplasia or carcinoma in situ, random biopsies are recommended.

Attempts have been made to develop various methods for in vivo labeling of those early cancers. Intravesical instillations of methylene blue have been abandoned because of the high rate of false negative results (70 to 85%).

Previously the detection of carcinoma in situ and dysplasia after Photofrin systemic injections was described. Disadvantages of this technique however are skin photosensitization for 24 hours, the requirement of administration of the drug 48 hours prior to the investigation, the point monitoring system that limits the detection area to 600 µm at a time and the diagnostic equipment itself, which must be handled by a physicist.

Aminolevulinic acid (ALA) is an other photosensitizer that can be used to tag bladder tumors. ALA can be administered intravesically, thereby eliminating systemic side effects. First reports were presented by Kriegmair et al. (J. Urol. 155, 105-109 (1996)). Six areas of carcinoma in situ were correctly diagnosed but 80% of false positive fluorescence areas were observed. More recently, Jichlinski et al. (Urol. Res. 25 suppl. 1, 3-6 (1997)) reported with the same technique a sensitivity and specificity to detect CIS of 83% and 81%, respectively. However, using exactly the same method, D'Hallewin et al. (Am. Clin. J. Oncol. 21(3), 223-225 (1998)) were not able to reproduce these data. The sensitivity to detect CIS was 94% but with a specificity of only 54%.

With the currently used fluorescence diagnostic techniques, the sensitivity to detect CIS is much higher than with the naked eye, but the relative lack of specificity is responsible for a high amount of false positive results.

In view of the above it is the object of the invention to provide a new way of diagnosing carcinoma in general, carcinoma of the urothelium in particular and specifically CIS without the drawbacks of the known methods described above.

This is achieved according to the invention by using hypericin as a diagnostic agent.

Hypericin is the hexahydroxydimethyl derivative of phenantroperylene dione and is one of the major photosensitizing constituents present in Hypericum extracts. The extract of Hypericum is used in the treatment of mild to moderately depressive disorders, especially in German speaking countries.

Hypericin shows a bright red fluorescence when excited. The photodynamic characteristics of the compound on human cell lines have been shown in vitro (Hadjur et al., J. Photochem. Photobiol. B. Biol. 27, 139-146 (1995); Couldwell et al., Neurosurgery 35, 705-709 (1994); Zhang et al., Neurosurgery 38, 705-709 (1996); Zhang et al., Cancer Lett. 96, 31-35 (1995); and VanderWerf et al., Laryngoscope 106, 479-483 (1996)). Vandenbogaerde et al. (Anticancer Res. 16, 1619-1626 (1996)) reported the selective accumulation and antitumoral activity of photosensitized hypericin in A431 cell xenografts in athymic nude mice. The accumulation in the tumor in this case took place after hypericin was illuminated.

In addition, it was demonstrated that hypericin exhibited in vitro a differential phototoxicity against several human tumor cell lines depending on the uptake of the compound by the cells (Vandenbogaerde et al., J. Photochem. Photobiol. B. 38, 136-142 (1997)). Recently a first clinical study was published using hypericin topically as photochemotherapeutic agent in squamous cell and basal cell carcinoma (Alecu et al., Anticancer Res. 18, 4651-4654 (1998)). For this hypericin was injected into the tumor and illuminated afterwards.

The diagnostic potential of hypericin however, has not yet been explored. The present inventors were the first to evaluate the potential use of hypericin as a selective tumor marker, in particular for bladder cancer. Surprisingly, it was found that hypericin was capable of concentrating very specifically into the tumor. Except for the tumor no other tissues showed fluorescence after illumination thus demonstrating the specificity. The advantage of this is that the amount of false positives will be lower than with the known markers.

The diagnostic preparation of the invention comprises hypericin and a suitable carrier or excipient and preferably takes the form of an instillation fluid. The instillation fluid is brought into the bladder and left there for a predetermined period of time. After removal of the fluid, the bladder can be illuminated with light that excites hypericin, e.g. blue light.

When excited the hypericin that is accumulated in the bladder carcinoma will emit orange-red fluorescence of 600-640 nm. This fluorescence can be detected with slightly modified lenses to enhance the blue/red contrast.

If desired the diagnosis can be coupled to local treatment by longer illumination times. Because the specificity of hypericin is so high, the treatment can also be highly specific and localised to the actual place of the tumor. Treatment without direct preceding diagnosis is also part of the invention.

The example will show that hypericin has various advantages over the commonly used ALA. In a study with 29 patients it was found that the use of hypericin for diagnosis according to the invention leads to a specificity in case of instillations of 98% with a negative predictive value (probability of a patient not having the disease when the test is negative) of 91%. The sensitivity after hypericin instillations is comparable to ALA.

It is interesting to note that in 7 out of the 10 false negative CIS biopsies, severe desquamation was noted, with only a few cells remaining to make the diagnosis. Diagnosis with ALA results in bright fluorescence in severely infectious eroded areas, as well as in case of desquamated CIS.

Fluorescence induced by ALA is rapidly bleached out and exposure to blue light has to be limited to short illumination periods in order to inspect the whole bladder. Hypericin induced fluorescence on the contrary is very stable, and the bladder can be fully inspected during the whole procedure.

An other advantage of hypericin as compared to ALA is the very slow fluorescence decay as a result of which patients can be instilled up to 16 hours prior to endoscopic inspection.

According to the invention hypericin can be used in lower concentrations than are used for other applications such as the treatment of depression or phytotoxicity. Typically, the amount to be administered for diagnosis according to the invention, is about a tenfold lower than the oral dose of total hypericin used to treat depression and approximately a 100 times lower than the phototoxic dose after oral administration of the Hypericum extract.

According to the invention it is advised to use 25 to 100 ml, preferably 50 ml of an instillation solution (for example plasma proteins in an isotonic buffered solution (pH 7.4)) which will be instilled for 2 to 4 hours, preferably 2 hours with a hypericin concentration of 1 to 10 µM. The total amount of hypericin instilled lies between 25 and 250 µg.

Hypericin is a lipophilic molecule and will therefore not readily dissolve in an aqueous medium. Therefore hypericin is preferably adsorbed to a protein, in particular albumin or a plasma protein preparation for solubilisation. In addition other solubilisation means can be used, such as cyclodextrin or liposomes.

Hypericin (as salt) for use in the invention can be either isolated from Hypericum species (e.g. H.perforatum) or synthetically prepared.

The term "hypericin" as used in this application is intended to encompass hypericin (as salt) itself, but also modified versions (analogues) or derivatives thereof. A preferred modification is a modification that leads to a higher hydrophilicity, thus facilitating dissolution of the compound in an aqueous medium. Such modifications can be formation of ethers or esters on the phenols or substitution of the aromatic rings.

The present invention will be further elucidated in the following example, which is given for illustration purposes only.

### EXAMPLE

### Diagnosis of carcinoma in the bladder

The instillation solution was prepared as follows. 1 to 10 mM hypericin in absolute ethanol was diluted a thousand fold in a 1% plasma protein solution and sterile membrane filtered.

29 patients were included in the study. 22 presented with visible exophytic transitional cell carcinoma. One patient had interstitial cystitis. 7 patients had repeated positive urinary cytology, without detectable lesions at cystoscopy, sonography or urogram. Random biopsies previously performed without hypericin instillation had been negative in 2 of them.

Instillation time varied between 1 and 4 hours (1, 2, 3 and 4 hours). At 2 to 24 hours after instillation, the patient were examined with white light cystoscopy, followed by blue light excitation. The system used for fluorescence diagnostics was developed by Storz company (D Light system). It consists of a small light source, with a Xenon arc lamp with a corresponding band pass filter (380-450 nm). By switching the foot pedal, the filter is placed or removed and blue or white light is produced, respectively. A conventional cystoscope is used for endoscopic examination, but the telescopes (0° and 30°) are provided with a long wavelength pass filter (>520 nm) to cut off the reflected blue excitation light without blocking the red fluorescence light. The telescopes are compatible with all Storz endoscopes.

Biopsies were performed from fluorescent positive and negative areas.

The instillation did not provoke any side effects nor symptoms.

All papillary lesions showed red fluorescence. Effective bright light fluorescence was obtained with 50 ml of a 2µM (or higher) solution.

Instillation times of 1 hour did not provoke any visible red fluorescence and minimum of 2 hours is required. The brightest fluorescence could be observed after 3 to 4 hours.

Fluorescence could be observed as early as 2 hours after instillation. The same intensity was observed after 12 to 16 hours, but was much reduced after 24 hours.

Carcinoma in situ could be correctly diagnosed in all of the patients with a suspicious cytology, without detectable tumor. 66 biopsies confirmed the presence of carcinoma in situ (Table 1). 56 of those biopsies had shown red fluorescence at cystoscopy.

**Table 1**

| | | | | |
|---|---|---|---|---|
| Red fluorescence (+) and blue fluorescence (-) with regard to pathology | | | | |
| CIS = carcinoma in situ | | | | |
| NL = normal transitional cell epithelium | | | | |
| NA = not applicable | | | | |

| | + | - | sensitivity | specificity |
|---|---|---|---|---|
| CIS | 56 | 10 | 85% | NA |
| NL | 2 | 97 | NA | 98% |

The sensitivity is the chance that the test will give a positive result in case a pathology is present and is obtained by dividing the amount of exact positives by the total of exact positives and false negatives. The specificity is the chance that a malignancy is present when the test is positive and is obtained by dividing exact negative by the sum of exact negatives and false positives.

## Claims

1. Use of hypericin or analogues or derivatives thereof for the preparation of a diagnostic composition for diagnosing carcinoma.

2. Use as claimed in claim 1 wherein the carcinoma originates from urothelium.

3. Use as claimed in claim 2 wherein the carcinoma is carcinoma of the bladder.

4. Use as claimed in claim 3 wherein the carcinoma is carcinoma in situ of the bladder.

5. Diagnostic preparation for diagnosing carcinoma, comprising a suitable amount of hypericin or analogues or derivatives thereof in a suitable carrier or excipient.

6. Instillation preparation for the diagnosis of carcinoma originating from the urothelium, comprising a suitable amount of hypericin or analogues or derivatives thereof in a suitable instillation fluid.

7. Instillation preparation as claimed in claim 6, wherein the suitable amount of hypericin is 1 to 10 µM.

8. Instillation preparation as claimed in claims 6 and 7, wherein the hypericin is adsorbed to a protein.

9. Instillation preparation as claimed in claim 8, wherein the protein is albumin or a plasma protein preparation.

10. Instillation preparation as claimed in claims 6-9 which is for use in diagnosing bladder carcinoma.

11. Instillation preparation as claimed in claims 6-9 which is for use in diagnosing carcinoma in situ.

12. Method for preparing an instillation preparation as claimed in claims 6-11, comprising the steps of:
a) solubilisation of hypericin in an aqueous buffer to obtain a hypericin solution; and
b) sterilisation of the solution thus obtained.

13. Method as claimed in claim 12, wherein the hypericin is solubilised by adsorption thereof to a protein, in particular albumin or a plasma protein preparation.

14. Method as claimed in claim 13, wherein the hypericin is solubilised by means of cyclodextrin or encapsulation in liposomes.

15. Method for diagnosing carcinoma, comprising contacting the site to be diagnosed for a suitable period of time with a suitable amount of a hypericin preparation, removing the preparation after lapse of the suitable period of time, illuminating the site with light of a suitable wavelength to excite the hypericin that has accumulated in the carcinoma to be diagnosed and detecting the fluorescence produced by the excited hypericin.

16. Method for diagnosing bladder cancer, comprising instilling in the bladder of a person to be diagnosed for a suitable period of time a suitable amount of an instillation preparation as claimed in claims 6-9, removing the instillation preparation after lapse of the suitable period of time, illuminating the bladder with light of a suitable wavelength to excite the hypericin that has accumulated in the carcinoma to be diagnosed and to detect fluorescence produced by the excited hypericin.

17. Method as claimed in claim 16, wherein the suitable period of time is at least 2 hours.

18. Method as claimed in claim 16 or 17, wherein the suitable amount is 30-70 ml, preferably 50 ml.

19. Method as claimed in claims 16-18, wherein the light is blue light.

20. Use of hypericin in the preparation of a therapeutical composition for the treatment of carcinoma, in particular carcinoma of urothelium, more in particular carcinoma of the bladder.
